# EUROPÄISCHE PATENTSCHRIFT

(11) **EP 0 715 835 B1**
(45) Veröffentlichungstag und Bekanntmachung des Hinweises auf die Patenterteilung: **14.05.2003**
(21) Anmeldenummer: 95101897.7
(22) Anmeldetag: 13.02.1995
(51) Int. Cl.: A61F 2/36

(54) **Verankerungsschaft für eine Gelenkendoprothese**
Anchoring shaft for a joint endoprosthesis
Tige d'ancrage pour une endoprothèse d'articulation

(30) Priorität: 09.11.1994 CH 335594
(43) Veröffentlichungstag der Anmeldung: 12.06.1996
(73) Patentinhaber: Intraplant AG, CH-6330 Cham (CH)
(72) Erfinder: Kuoni, Xaver, 8952 Schlieren (CH)
(74) Vertreter: Patentanwälte Schaad, Balass, Menzl & Partner AG

(56) Entgegenhaltungen:
- EP-A- 0 209 516
- EP-A- 0 378 044
- EP-A- 0 390 768
- WO-A-83/02555
- DE-A- 2 839 092

## Beschreibung

Die Erfindung betrifft einen Verankerungsschaft für eine Glenkendoprothese, insbesondere Hüftgelenkprothese, nach dem Oberbegriff des Patentanspruchs 1.

Ein Verankerungsschaft dieser Art ist beispielsweise bekannt aus EP-A-0 390 768. In EP-A-0 427 902 ist ein Schaft einer Femurkopfprothese beschrieben, der sich von einem distalen Ende nach allen Seiten konisch erweitert. Der Querschnitt des Schaftes ist in der distalen, unteren Hälfte quadratisch und der Schaft geht in der proximalen, oberen Hälfte in eine abgerundete rechteckige oder eine ovale Form über. Die quadratische Form zum distalen Ende wird hier deshalb bevorzugt, da dadurch halbrunde Zwischenräumen entstehen, in welchen sich spongioses Knochengewebe bildet, bzw. eine Revaskularisierung des Knochens stattfindet. Die Kanten in der unteren Schafthälfte sind mit sägezahnartigen Zähnen versehen, deren Zahnspitzen in der Kante liegen, d.h. leicht zurückversetzt sind. Die Zähne sind kurz und mit einer sehr geringen Zahntiefe ausgebildet (vergleiche Schaftquerschnitt in Fig. 3), so dass der ursprüngliche quadratische Querschnitt mindestens annähernd erhalten geblieben ist. Die Zähne von nach medial zeigenden Zahnreihen können so ausgebildet, dass sie eine gewisse Schneidwirkung entfalten, während die Zähne der anderen Zahnreihen so ausgebildet sind, dass sie nicht schneiden, sondern nur die Knochensubstanz verdrängen. Trotz dieser Zähne wird demnach die abstützende Wirkung des quadratischen Schaftes entlang der Kanten beibehalten.

Der Erfindung liegt daher die Aufgabe zugrunde, einen Verankerungsschaft der vorgenannten Art zu schaffen, welcher mühelos in den vorbereiteten Knochenhohlraum eingetrieben werden kann bei einer möglichst grossen Schonung der Knochensubstanz. Ferner soll eine stabile Verankerung im Knochen gegeben sein, so dass keine Lockerung durch die normale Beanspruchung der Gelenke erfolgt.

Diese Aufgabe wird durch einen Verankerungsschaft mit den Merkmalen des Patentanspruchs 1 gelöst.

Die Erfindung weist den grossen Vorteil auf, dass die Schneidflächen der über den Kern des Verankerungsschaftes vorstehenden und in Längsrichtung ausgerichteten sägezahnartigen Rippen verhältnismässig leicht in den Knochen eingetrieben werden können. Die Rippen des Verankerungsschaftes sind direkt von der Knochensubstanz umgeben, so dass eine ausgezeichnete Verankerung gewährleistet ist. Durch die in die Knochensubstanz eingreifenden Zähne mit ihren steilen Zahnflanken nach distal wird ein Einsinken des Verankerungsschaftes in den Knochen hinein wirksam verhindert. Da die Rippen über ihre ganze Länge eine gleichbleibende Dicke aufweisen, entstehen beim Eintreiben keine seitliche Spaltkräfte, so dass der Knochen beim Eintreiben nicht aufgesprengt wird.

Weitere Vorteile der vorliegenden Erfindung ergeben sich aus den abhängigen Patentansprüchen und aus der nachfolgenden Beschreibung. Dort wird die Erfindung anhand eines in den Zeichnungen dargestellten Beispiels näher erläutert. Es zeigt:
- Fig. 1: eine Seitenansicht eines Verankerungsschaftes für eine Gelenkendoprothese, und
- Fig. 2: einen Querschnitt längs der Linie A-A in Figur 1.

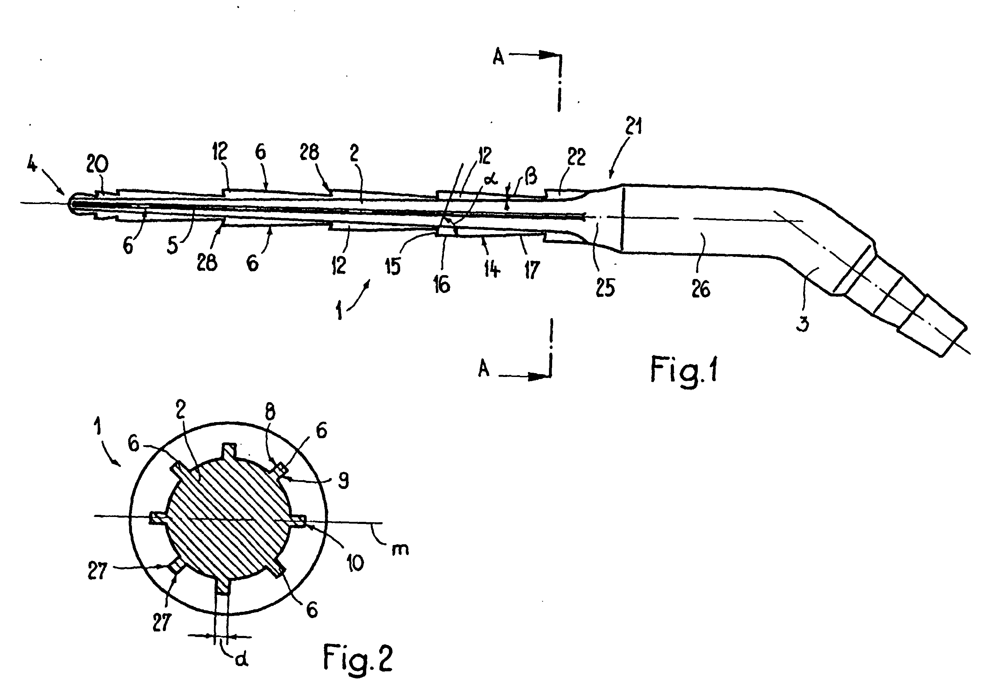

In den Figuren ist ein Verankerungsschaft 1 einer Hüftgelenkprothese mit einem geraden Kern 2 und einem angewinkelten Befestigungsteil 3 für einen kugelförmigen Gelenkkopf gezeigt. Der Kern 2 hat einen kreisrunden Querschnitt (vergleiche Figur 2) und ist gegen das distale Ende 4 konisch verjüngt ausgebildet. Am Umfang des Kerns 2 sind in Längsrichtung 5 verlaufende, radial nach aussen abstehende Rippen 6 vorgesehen, die je zwei parallele Seitenflächen 8 und 9 haben und somit eine über die ganze Länge gleichbleibende Dicke d aufweisen (Fig. 2). Die Stirnseite 10 jeder Rippe 6 ist eben und im Querschnitt (Fig. 2) rechtwinklig zur jeweiligen Mittelebene m durch die Rippe 6. Die Rippe 6 weist ferner in ihrem mittleren Bereich eine Reihe von vier sägezahnartigen Zähnen 12 auf, die zum Teil unterschiedlich lang sind. Jeder Zahn 12 hat eine lange Zahnflanke 14 und eine kurze oder steile Zahnflanke 15, welche einen Winkel α von annähernd, d.h. etwas kleiner, als 90° einschliessen. Die lange Zahnflanke 14 weist einen kürzeren zum Umfang des Kerns 2 parallelen Teil 16 und einen dazu angewinkelten längeren Teil 17 auf, der einen spitzen Winkel β von kleiner als etwa 30° zur Längsrichtung am Umfang des Kerns 2 einschliesst. Zum distalen Ende 4 hin ist ein kurzer Zahn 20 an jeder Rippe 6 vorgesehen, der dieselbe Form wie die Zähne 12 aufweist. Zum proximalen Ende 21 des Kerns 2 hin weist die Rippe 6 ferner ein Zahnstück 22 auf, das ebenfalls dieselbe Form wie die Zähne 12 aufweist, jedoch grösser ist und etwa nach einem Drittel der normalen Länge in ein konisch ausgeweitetes Übergangsstück 25 des Kerns 2 übergeht. Dieses Übergangsstück 25 führt dann abgerundet in einen zylindrischen Teil 26 über, an welchem der angewinkelte Befestigungsteil 3 anschliesst. Der zylindrische Teil 26 hat einen grösseren Durchmesser als derjenige des Kerns 2.

Die Kanten 27 zwischen den Seitenflächen 8 und 9 und der Stirnseite 10 jeder Rippe 6 sind scharf, und bilden somit eine Schneidkante (siehe Fig. 2). Dies gilt zumindest für die kurze Zahnflanke 15 der Zähne 12, 20 und 22, kann jedoch auch für die lange Zahnflanke 14 dieser Zähne vorgesehen sein. Auf jedem Fall sind auch die Zahnspitzen 28, die sich zwischen der langen Zahnflanke 14 und der kürzen Zahnflanke 15 befindet, scharf ausgebildet (siehe Fig. 1).

Der Verankerungsschaft 1 ist normalerweise einstückig aus einem an sich für solche Prothesen bekannten Werkstoff hergestellt. Die kurzen oder steilen Zahnflanken 15 der Rippen 6 verhindern ein Einsinken des Verankerungsschaftes 1 in die Knochenmasse, was bei den normalen Belastungen auf das Gelenk von grosser Bedeutung ist. Ferner wird durch die relativ schmalen Rippen 6 eine rasche Stabilisierung des Verankerungsschaftes 1 im Knochen erreicht, nachdem die Gelenkendoprothese operativ eingesetzt worden ist. Der Verankerungsschaft 1 mit den Rippen 6 ist jedoch nicht nur für eine Hüftgelenkprothese sondern auch für andere Gelenkendoprothesen wie Ellbogengelenkprothesen oder Fussgelenkprothesen geeignet. Ferner ist die einfache Gestaltung des Verankerungsschaftes 1 mit den Rippen 6 besonders vorteilhaft als Ersatz für bestehende Gelenkendoprothesen anwendbar, da das Knochengewebe durch die spezielle Ausbildung der Schneidkanten wie die Kanten 27 und die Zahnspitzen 28 beim Eintreiben des Verankerungsschaftes 1 in den im Knochen ausgeräumten Hohlraum nicht aufgesprengt wird.

## Patentansprüche

1. Verankerungsschaft (1) für eine Gelenkendoprothese, insbesondere Hüftgelenkprothese, mit einem geraden, sich in Richtung einer Längsachse erstreckenden Kern (2), welcher gegen das distale Ende (4) hin verjüngt ausgebildet ist, einen bezüglich seiner Längsachse symmetrischen Querschnitt aufweist und mit in seiner Längsrichtung (5) ausgerichteten Reihen von vom Kern (2) abstehenden, sägezahnartigen Zähnen (12), die mit ihrer steilen Zahnflanke (15) nach distal weisen, versehen ist wobei die Reihen von Zähnen (12, 20, 22) mit Abstand vom distalen Ende (4) im distalen Endbereich des Kerns beginnen und sich gegen das proximale Ende (21) des Kerns (2) hin erstrecken, und wobei die Reihen von Zähnen an vom Kern (2) radial vorstehenden, in Längsrichtung (5) des Kerns (2) verlaufenden Rippen (6) ausgebildet sind, die über ihre Länge eine gleichbleibende Dicke (d) aufweisen, **dadurch gekennzeichnet, dass** die Zähne mit einer langen Zahnflanke (14) radial nach außen weisen, wobei die lange und die steile Zahnflanke einen Winkel von etwas kleiner als 90° einschliessen und wobei die lange Zahnflanke (14) einen spitzen Winkel von kleiner als 30° zur Längsrichtung am Umfang des Kerns einschliesst, und dass die Rippen (6) einen rechteckigen Querschnitt aufweisen.

2. Verankerungsschaft nach Anspruch 1, **dadurch gekennzeichnet, dass** die steile Zahnflanke (15) kurz ist.

3. Verankerungsschaft nach Anspruch 1 oder 2, **dadurch gekennzeichnet, dass** der Kern (2) des Verankerungsschaftes (1) einen kreisrunden Querschnitt aufweist.

4. Verankerungsschaft nach einem der Ansprüche 1 bis 3, **dadurch gekennzeichnet, dass** die Rippen (6) gleichmässig am Umfang des Kernes (2) verteilt sind.

5. Verankerungsschaft nach einem der Ansprüche 1 bis 4, **dadurch gekennzeichnet, dass** das distale Ende (4) des Kernes (2) gerundet ausgebildet ist.

6. Verankerungsschaft nach einem der Ansprüche 1 bis 5, **dadurch gekennzeichnet, dass** die Zahnspitzen (28) der Zähne (12, 20, 22) und zumindest die Kanten (27) der kurzen Zahnflanken (15) scharf ausgebildet sind.

## Claims

1. Anchoring shaft (1) for a joint endoprosthesis, in particular a hip joint prosthesis, with a rectilinear core (2) which extends in the direction of a longitudinal axis, is designed tapering towards the distal end (4), has a cross section which is symmetrical with respect to its longitudinal axis and is provided with series, oriented in its longitudinal direction (5), of sawtooth-like teeth (12) which protrude from the core (2) and point in the distal direction with their steep tooth flank (15), the series of teeth (12, 20, 22) beginning at a distance from the distal end (4) in the distal end region of the core and extending towards the proximal end (21) of the core (2), and the series of teeth being formed on ribs (6) which protrude radially from the core (2), run in the longitudinal direction (5) of the core (2) and have a uniform thickness (d) over their length, **characterized in that** the teeth point radially outwards with a long tooth flank (14), the long tooth flank and the steep tooth flank enclosing an angle of slightly less than 90°, and the long tooth flank (14) enclosing an acute angle of less than 30° with respect to the longitudinal direction at the periphery of the core, and **in that** the ribs (6) are rectangular in cross section.

2. Anchoring shaft according to Claim 1, **characterized in that** the steep tooth flank (15) is short.

3. Anchoring shaft according to Claim 1 or 2, **characterized in that** the core (2) of the anchoring shaft (1) has a circular cross-section.

4. Anchoring shaft according to one of Claims 1 to 3, **characterized in that** the ribs (6) are distributed uniformly on the circumference of the core (2).

5. Anchoring shaft according to one of Claims 1 to 4, **characterized in that** the distal end (4) of the core (2) is designed rounded.

6. Anchoring shaft according to one of Claims 1 to 5, **characterized in that** the tooth points (28) of the teeth (12, 20, 22) and at least the edges (27) of the short tooth flanks (15) are designed sharp.

## Revendications

1. Tige d'ancrage (1) pour une endoprothèse articulaire, en particulier une prothèse articulaire de la hanche, avec un noyau (2), allongé dans la direction d'un axe longitudinal, lequel s'amincit vers l'extrémité distale (4), présente une section transversale symétrique par rapport à son axe longitudinal et est pourvu de rangées alignées dans sa direction longitudinale (5) de dents en forme de dents de scie (12), saillant depuis le noyau (2), dont la facette dentaire abrupte (15) est dirigée vers l'extrémité distale, les rangées de dents (12, 20, 22) ayant leur origine dans la partie d'extrémité distale du noyau à une certaine distance de l'extrémité distale (4) et se prolongeant vers l'extrémité proximale (21) du noyau (2), et les rangées de dents étant réalisées sur des nervures (6) saillant radialement à partir du noyau (2) s'étendant dans la direction longitudinale (5) du noyau (2), lesquelles présentent sur toute leur longueur la même épaisseur (d), **caractérisée en ce que** les dents avec une facette dentaire allongée (14) sont dirigées radialement vers l'extérieur, les facettes dentaires allongées et abruptes formant un angle d'un peu moins de 90° et la facette dentaire allongée (14) formant un angle aigu de moins de 30° par rapport à la direction longitudinale au niveau de la circonférence du noyau, et **en ce que** les nervures (6) présentent une section transversale rectangulaire.

2. Tige d'ancrage selon la revendication 1, **caractérisée en ce que** la facette dentaire abrupte (15) est courte.

3. Tige d'ancrage selon la revendication 1 ou 2, **caractérisée en ce que** le noyau (2) de la tige d'ancrage (1) présente une section transversale circulaire.

4. Tige d'ancrage selon l'une des revendications 1 à 3, **caractérisée en ce que** les nervures (6) sont réparties uniformément sur la circonférence du noyau (2).

5. Tige d'ancrage selon l'une des revendications 1 à 4, **caractérisée en ce que** l'extrémité distale (4) du noyau (2) a une forme arrondie.

6. Tige d'ancrage selon l'une des revendications 1 à 5, **caractérisée en ce que** les pointes (28) des dents (12, 20, 22) et au moins les arêtes (27) des facettes dentaires courtes (15) sont acérées.
